# EUROPÄISCHE PATENTSCHRIFT

(11) **EP 4 703 420 B1**
(45) Veröffentlichungstag und Bekanntmachung des Hinweises auf die Patenterteilung: **04.11.2026**
(21) Anmeldenummer: 24198044.0
(22) Anmeldetag: 03.09.2024
(51) Int. Cl.: C08K 5/00, C07C 69/82, C07C 69/608

(54) **WEICHMACHERZUSAMMENSETZUNG ENTHALTEND C4- BIS C9-ALKYLESTER DER 1,2,4-CYCLOHEXANTRIPROPIONSÄURE UND DIBUTYLTEREPHTHALAT ODER DIPENTYLTEREPHTHALAT**
PLASTICIZER COMPOSITION COMPRISING C4- TO C9-ALKYL ESTERS OF 1,2,4-CYCLOHEXANETRIPROPIONIC ACID AND DIBUTYLTEREPHTHALATE OR DIPENTYLTEREPHTHALATE
COMPOSITION PLASTIFIANTE CONTENANT DES ESTERS ALKYLIQUES C4 À C9 DE L'ACIDE 1,2,4-CYCLOHEXANETRIPROPIONIQUE ET DU POLYTÉRÉPHTALATE DE DIBUTYLE OU DU DIOXATÉRÉPHTHALATE

(43) Veröffentlichungstag der Anmeldung: 04.03.2026
(73) Patentinhaber: Evonik Oxeno GmbH & Co. KG, 45772 Marl (DE)
(72) Erfinder: GRASS, Michael, 45721 Haltern am See (DE); RUSEK, Monika, 45149 Essen (DE); VAN EICKELS, Michael, 45657 Recklinghausen (DE)
(74) Vertreter: Evonik Patent Association

(56) Entgegenhaltungen:
- EP-A1- 3 838 886
- EP-A1- 3 838 888
- WO-A1-2019/236342
- RU-A- 2015 156 846

## Beschreibung

Gegenstand der Erfindung ist eine Weichmacherzusammensetzung enthaltend eine Verbindung der Formel (1) 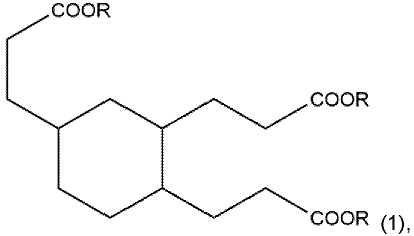 wobei die drei Alkylgruppen R jeweils 4 bis 9 Kohlenstoffatome aufweisen, und Dibutylterephthalat oder Dipentylterephthalat.

Die erfindungsgemäßen Verbindungen nach der Formel (1) sind C4- bis C9-Alkylester der 1,2,4-Cyclohexantripropionsäure. Diese Ester der 11,2,4-Cyclohexantripropionsäure sind grundsätzlich bekannt und beispielsweise in der EP 3 838 888 A1 beschrieben worden. Dort wurde auch erwähnt, dass diese Ester der 1,2,4-Cyclohexantripropionsäure als Weichmacher für Polymere eingesetzt werden können.

Auch wenn die Trialkylester der 1,2,4-Cyclohexantripropionsäure bekannt und ihr Einsatz als Weichmacher erwähnt worden ist, besteht ein kontinuierlicher Bedarf an einer Verbesserung der Eigenschaften beim Einsatz als Weichmacher. Je nach Anwendung können die Anforderungen an den einzusetzenden Weichmacher variieren.

Der vorliegenden Erfindung lag nun die Aufgabe zugrunde, eine Weichmacherzusammensetzung bereitzustellen, die die Eigenschaften der C4- bis C9-Alkylester der 1,2,4-Cyclohexantripropionsäure weiter verbessert.

Diese Aufgabe wird gelöst durch die Weichmacherzusammensetzung nach Anspruch 1. Gegenstand der Erfindung ist demnach eine Weichmacherzusammensetzung enthaltend eine Verbindung der Formel (1) 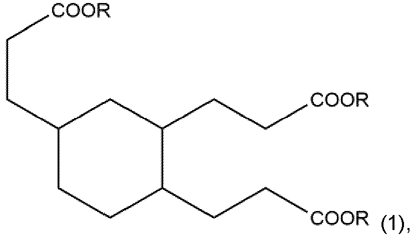 wobei die drei Alkylgruppen R jeweils 4 bis 9 Kohlenstoffatome aufweisen, und Dibutylterephthalat oder Dipentylterephthalat. Bevorzugte Ausführungsformen der vorliegenden Erfindung sind in den Unteransprüchen angegeben.

Die erfindungsgemäßen Verbindungen nach Formel (1) sind folglich C4- bis C9-Alkylester der 1,2,4-Cyclohexantripropionsäure. Die drei Alkylgruppen R können dabei jeweils unabhängig voneinander aus der Gruppe, bestehend aus einer normalen Butylgruppe, einer Isobutylgruppe, einer normalen Pentylgruppe, einer 2-Methylbutylgruppe, einer 3-Methylbutylgruppe, einer normalen Hexylgruppe, einer Isohexylgruppe, einer normalen Heptylgruppe, einer Isoheptylgruppe, einer normalen Ocylgruppe, einer Isooctylgruppe, einer normalen Nonylgruppe oder einer Isononylgruppe, ausgewählt werden. Unter einer Isononylgruppe wird im Sinne der vorliegenden Erfindung eine Mischung aus linearen und verzweigten primären C9-Alkylgruppen verstanden, Entsprechendes gilt für die Begriffe Isohexylgruppe, Isoheptylgruppe und Isodecylgruppe..

In einer bevorzugten Ausführungsform der vorliegenden Erfindung weisen die drei Alkylgruppen R der längerkettigen Trialkylester der 1,2,4-Cyclohexantripropionsäure jeweils die gleiche Anzahl an Kohlenstoffatomen auf. Das bedeutet, dass alle drei Alkylgruppen R 4, 5, 6, 7, 8 oder 9 Kohlenstoffatome aufweisen, aber nicht, dass die drei Alkylgruppen immer identisch sein müssen. Es können auch gleichzeitig unterschiedliche Isomere der jeweiligen Alkylgruppe vorliegen, beispielsweise bei der C5-Alkylgruppe 2-Methylbutyl- und n-Pentylgruppen.

Es ist im Rahmen der vorliegenden Erfindung besonders bevorzugt, dass die Verbindung nach Formel (1) in der Weichmacherzusammensetzung ein Tripentylester der 1,2,4-Cyclohexantripropionsäure oder ein Triisononylester der 1,2,4-Cyclohexantripropionsäure ist. Unter den Begriff Tripentylester der 1,2,4-Cyclohexantripropionsäure fallen Trialkylester der 1,2,4-Cyclohexantripropionsäure, bei denen jede der drei Alkylgruppen eine 2-Methylbutylgruppe, eine 3-Methylbutylgruppe oder eine n-Pentylgruppe ist. Vorzugsweise beträgt der Anteil von n-Pentylgruppen mindestens 55 % und maximal 95% bezogen auf alle (= 100%) der Pentylgruppen im Tripentylester der 1,2,4-Cyclohexantripropionsäure. Unter den Begriff Triisononylester der 1,2,4-Cyclohexantripropionsäure fallen ebenso Mischester, die unterschiedliche C9-Alkylgruppen (Isomere der Nonylgruppe) enthalten. Grund dafür ist die Herstellung mittels kommerziell erhältlichem Isononanol (z. B. von der Evonik Oxeno Gmbh & Co. KG), das typischerweise eine Mischung unterschiedlicher C9-Alkohole (linear und verzweigt) darstellt.

Die Herstellung der erfindungsgemäßen längerkettigen Trialkylester der 1,2,4-Cyclohexantripropionsäure kann über die Umesterung des Trimethylesters der 1,2,4-Cyclohexantripropionsäure mit einem Alkohol mit 4 bis 9 Kohlenstoffatomen erfolgen oder über die direkte Veresterung der 1,2,4-Cyclohexantripropionsäure mit einem Alkohol mit 4 bis 9 Kohlenstoffatomen. Sollen der Tripentyl- oder der Triisononylester der 1,2,4-Cyclohexantripropionsäure hergestellt werden, sind Pentanol oder Isononanol als Alkohol einzusetzen. Der Alkohol wird dabei im Überschuss eingesetzt, d. h. in einer Menge von mehr als 3 Mol pro Mol Esterkomponente, vorzugsweise 3,75 bis 5,25 mol Alkohol pro Mol Esterkomponente. Die Umesterung und die direkte Veresterung werden bevorzugt an einem sauren Katalysator durchgeführt, beispielsweise anorganische oder organische Brönsted- oder Lewis-Säuren.

Die Weichmacherzusammensetzung enthält zusätzlich zu den Verbindungen nach Formel (1) Dibutylterephthalat, abgekürzt auch DBT, oder Dipentylterephthalat, abgekürzt auch DPT. Beide Weichmacher sind Terephthalsäureester und kommerziell erhältlich.

Die beiden Substanzen, d. h. der C4- bis C9-Alkylester der 1,2,4-Cyclohexantripropionsäure als Verbindung nach Formel (1) und Dibutylterephthalat oder Dipentylterephthalat, können in unterschiedlichen Mengen in der erfindungsgemäßen Weichmacherzusammensetzung vorhanden sein. Es sollte klar sein, dass die beiden Substanzen in einer Menge vorhanden sein müssen, bei der sie eine Wirkung entfalten, d. h. wo eine weichmachende Wirkung eintritt. In einer bevorzugten Ausführungsform der vorliegenden Erfindung liegen der C4- bis C9-Alkylester der 1,2,4-Cyclohexantripropionsäure als Verbindung nach Formel (1) und Dibutylterephthalat oder Dipentylterephthalat in einem Gewichtsverhältnis (C4- bis C9-Alkylester der 1,2,4-Cyclohexantripropionsäure als Verbindung nach Formel (1) : Dibutylterephthalat oder Dipentylterephthalat) von 1 : 99 bis 99 : 1, vorzugsweise 10 : 90 bis 90 : 10, besonders bevorzugt 30 : 70 bis 70 : 30 in der Weichmacherzusammensetzung vor.

Die erfindungsgemäße Weichmacherzusammensetzung der vorliegenden Erfindung kann zusätzlich ein epoxidiertes Öl oder einen epoxidierten Alkylester einer Fettsäure enthalten. Epoxidierte Öle oder entsprechende Ester können die thermische Stabilität und die mechanischen Eigenschaften verbessern.

Das epoxidierte Öl kann aus der Gruppe bestehend aus epoxidiertem Sojabohnenöl, epoxidiertem Rizinusöl, epoxidiertem Leinöl, epoxidiertem Palmöl, epoxidiertem Tallöl und Mischungen davon ausgewählt werden. Bevorzugt werden epoxidiertes Sojabohnenöl oder epoxidiertes Leinöl in der erfindungsgemäßen Weichmacherzusammensetzung eingesetzt. Besonders bevorzugt ist epoxidiertes Sojabohnenöl, das auch unter dem Akronym ESBO bekannt ist.

Die epoxidierten Fettsäureester können durch Umesterung der o.g. epoxidierten Öle mit Alkoholen im Bereich von 1 bis 10 Kohlenstoffatomen, bevorzugt 4 bis 9 Kohlenstoffatomen, hergestellt werden. Alternativ kann erst das natürliche Öl umgeestert und die Doppelbindungen der Fettsäure anschließend epoxidiert werden.

Das epoxidierte Öl oder die entsprechenden epoxidierten Fettsäurealkylester können in einer Menge von 1 bis 150 Gewichtsteilen bezogen auf 100 Gewichtsteile der Gesamtsumme aus C4- bis C9-Alkylester der 1,2,4-Cyclohexantripropionsäure als Verbindung nach Formel (1) und Dibutylterephthalat oder Dipentylterephthalat eingesetzt werden. Das epoxidierte Öl oder die epoxidierten Fettsäureester können auch in Mengen von 2 bis 125 Gewichtsteilen, 5 bis 100 Gewichtsteilen, 10 bis 80 Gewichtsteilen oder 20 bis 70 Gewichtsteilen jeweils bezogen auf 100 Gewichtsteile aus C4- bis C9-Alkylester der 1,2,4-Cyclohexantripropionsäure als Verbindung nach Formel (1) und Dibutylterephthalat oder Dipentylterephthalat in der Weichmacherzusammensetzung enthalten sein.

Die erfindungsgemäße Weichmacherzusammensetzung kann zusätzlich noch mindestens einen weiteren Weichmacher enthalten, der aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten mit Ausnahme der bereits vorhandenen Terephthalate, Isophthalaten, Phthalaten, Trimellitaten, Cyclohexandicarboxylaten und oligomeren oder polymeren Estern auf Basis von Adipin-, Bernstein- oder Sebacinsäure, ausgewählt wird.

In einer bevorzugten Ausführungsform der vorliegenden Erfindung enthält die Weichmacherzusammensetzung mindestens einen weiteren Weichmacher, der aus der Gruppe, bestehend aus Alkylbenzoaten, Alkylsulfonsäureestern des Phenols, Dialkyladipaten, Glycerinestern, C4-bis C6-Alkansäureester von Polyolen, acetylierten oder nicht acetylierten Citronensäuretrialkylestern, Glykoldibenzoaten, Trialkylestern der Trimellitsäure, Dialkylterephthalaten mit Ausnahme der bereits vorhandenen Terephthalate, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate, Dialkylestern der 1,2-, 1,3- oder 1,4-Cyclohexandicarbonsäure.

In einer weiterhin bevorzugten Ausführungsform wird der mindestens eine weitere Weichmacher, der in der erfindungsgemäßen Weichmacherzusammensetzung enthalten sein kann, aus der Gruppe, bestehend aus C8- bis C13-Alkylbenzoaten, C4- bis C10-Dialkyladipaten, Pentaerythrit-tetravalerat, acetylierten oder nicht acetylierten Citronensäuretrialkylestern mit C2- bis C9-Alkylgruppen, C4- bis C10-Trialkyltrimellitaten, C6- bis C9-Dialkylterephthalaten, C4- bis C13-Dialkylphthalaten, insbesondere C9-bis C13-Dialkylphthalaten und C4- bis C10-Dialkylestern der 1,2-, 1,3- oder 1,4-Cyclohexandicarbonsäure, ausgewählt.

Besonders bevorzugt werden von den Citraten Butyl- oder Pentyl- oder höhere Citrate eingesetzt, die eine Acetylgruppe aufweisen können. Darunter fallen Tributylcitrat, Tripentylcitrat, Tri-n-hexylcitrat, Tri-2-ethylhexylcitrat, Triisononylcitrat, Acetyltributylcitrat und Acetyltripentylcitrat. Von den C4- bis C10-Dialkyladipaten sind Diethylhexyladipat (DEHA) und Diisononyladipat (DINA) bevorzugt. Von den C6- bis C9-Dialkylterephthalaten ist Diethylhexylterephthalat (DEHT bzw. DOTP) bevorzugt. Von den C4- bis C10-Trialkyltrimellitaten sind Triethylhexyltrimellitat (TEHTM bzw. TOTM), und Triisononylterephthalat (TINTM) bevorzugt. Von den C4- bis C10-Dialkylestern der 1,4-Cyclohexandicarbonsäure sind der 1,4-Di-2-ethylhexylcyclohexandicarbonsäureester (1,4-DEHCH) und der 1,4-Diisononylcyclohexandicarbonsäureester (1,4-DINCH bzw. DINCD) bevorzugt. Von den C4- bis C10-Dialkylestern der 1,2-Cyclohexandicarbonsäure sind der 1,2-Di-2-ethylhexylcyclohexandicarbonsäureester (1,2-DEHCH) und der 1,2-Diisononylcyclohexandicarbonsäureester (1,2-DINCH) bevorzugt.

Ein weiterer Gegenstand der vorliegenden Erfindung ist eine Kunststoffzusammensetzung, die einen Kunststoff und die Weichmacherzusammensetzung, umfassend die C4- bis C9-Alkylester der 1,2,4-Cyclohexantripropionsäure als Verbindung nach Formel (1) und Dibutylterephthalat oder Dipentylterephthalat, enthält. Die Kunststoffzusammensetzung kann zudem das epoxidierte Öl und /oder ein epoxidierten Fettsäurealkylester und/oder mindestens einen zusätzlichen Weichmacher aus der oben genannten Liste enthalten.

Geeignete Kunststoffe sind polymere Substanzen, die vorzugsweise aus der Gruppe, bestehend aus PVC (Polyvinylchlorid), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikonen, ausgewählt werden.

In einer bevorzugten Ausführungsform ist der Kunststoff in der Weichmacherzusammensetzung ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat. Besonders bevorzugt ist hiervon PVC als Kunststoff in der erfindungsgemäßen Kunststoffzusammensetzung.

Die Menge an der erfindungsgemäßen Weichmacherzusammensetzung in der Kunststoffzusammensetzung beträgt vorzugsweise 5 bis 150 Gewichtsteile, bevorzugt 10 bis 120 Gewichtsteile, besonders bevorzugt 15 bis 110 Gewichtsteile und ganz besonders bevorzugt 20 bis 100 Gewichtsteile pro 100 Gewichtsteile des Kunststoffs.

Die Kunststoffzusammensetzung kann neben den erwähnten Inhaltsstoffen zusätzliche Additive enthalten. Beispiele für zusätzliche Additive sind rheologische Additive, mit denen die Viskosität der Kunststoffzusammensetzung verringert werden kann. Beispiele für bekannte rheologische Additive sind die unter den Handelsnamen VISCOBYK^{®}-5120, VISCOBYK^{®}-5130 und VISCOBYK^{®}-4041 erhältlichen Produkte. Die Additive können in einem Anteil von 1 bis 12, bevorzugt 2 bis 10 Gewichtsteilen pro 100 Gewichtsteilen PVC in der Kunststoffzusammensetzung enthalten sein.

Darüber hinaus kann die Kunststoffzusammensetzung einen oder mehrere Thermostabilisator(en) enthalten. Geeignete Thermostabilisatoren sind Bleisalze, Organozinnverbindungen, Barium/Zinkverbindungen, Cadmiumverbindungen oder Zinkverbindungen, Calcium/Zink-Stabilisatoren und organisch-basierte Stabilisatoren (sog. OBS). Bevorzugte Thermostabilisatoren sind Barium/Zinkverbindungen, Calcium/Zink-Stabilisatoren und organisch-basierte Stabilisatoren (sog. OBS). Der Anteil des oder der Stabilisatoren in der Kunststoffzusammensetzung beträgt vorzugsweise 1 bis 6 Gewichtsteile pro 100 Gewichtsteilen PVC.

Als weitere Additive können darüber hinaus auch Füllstoffe, Pigmente, Treibmittel und Gleitmittel in der Kunststoffzusammensetzung enthalten sein.

Die erfindungsgemäße Kunststoffzusammensetzung ist vorzugsweise Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Gegenstand der vorliegenden Erfindung ist deshalb auch die Verwendung der Kunststoffzusammensetzung in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

Die vorliegende Erfindung wird nachfolgend anhand von Beispielen illustriert. Die folgenden Beispiele sollen die Erfindung erläutern, ohne deren Anwendungsbreite, die sich aus der Beschreibung und den Patentansprüchen ergibt, einzuschränken.

### Beispiele

### Beispiel 1a - Herstellung von Cyclohexan-1,2,4-tripropionsäuretrimethylester (Me-Tc)

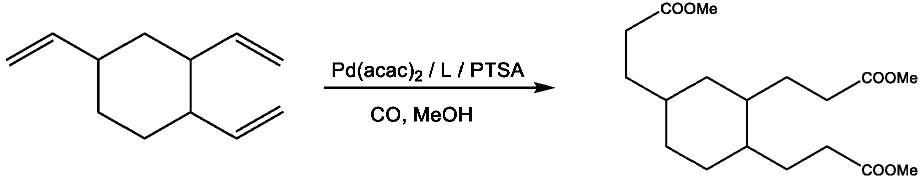

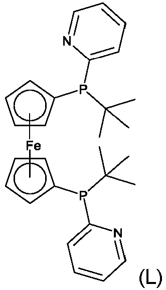

[Pd(acac)2] (15,2 mg, 0,1 mol%), der Katalysator L (103 mg, 0,4 mol%) und p-Toluolsulfonsäure (PTSA) (143 mg, 1,5 mol%) wurden unter einer Argonatmosphäre in einen 100-ml-Stahlautoklaven gegeben. Dann wurden MeOH (30 ml) und Trivinylcyclohexan (8,1 g, 50 mmol) mittels einer Spritze injiziert. Der Autoklav wurde dreimal mit CO gespült und anschließend mit einem CO-Druck von 40 bar beaufschlagt. Die Reaktion erfolgte über 10 h bei 110 °C. Danach wurde der Autoklav auf Raumtemperatur abgekühlt und entspannt. Das gewünschte Produkt wurde durch Destillation (165 °C bei 10⁻³ bar) aufgereinigt und mit ¹H-, ¹³C-NMR und HR-MS charakterisiert (15,6 g, 91% Ausbeute).

### Beispiel 1b - Herstellung von Cyclohexan-1,2,4-Tripropionsäure-tripentylester (MbPe-Tc)

Der nach Beispiel 1a hergestellte Trimethylester (Me-Tc) wurde in einer typischen Labor-Umesterungsapparatur mit 2-Methylbutanol und n-Pentanol (molares Verhältnis 7:93) zum Produkt MbPe-Tc umgeestert. Die Umesterungsapparatur besteht aus einem Glaskolben, der mit einem Rührer, Temperaturfühler, einer Destillationskolonne, Vakuumteiler und Destillationsaufbau ausgestattet ist.

Zunächst wurden 127 g 2-Methybutanol, 1690 g n-Pentanol und 1880 g M-Tc in der Umesterungsapparatur vorgelegt. Im Anschluss wurde die gesamte Apparatur mit Stickstoff gespült. und danach 2,72 g Tetra(n-butyl)titanat (TNBT) als Katalysator hinzugegeben. Danach wurde die Reaktion durch Aufheizen gestartet. Die Reaktionstemperatur betrug dabei 130 - 240°C, d. h. sie stieg während der Reaktion an. Methanol, das bei der Umesterung entstand, wurde über die Kolonne und Destillationsapparatur aus dem System entfernt (65°C Kopftemperatur). Die C5-Alkohole, welche in der Kolonne vom Methanol getrennt wurden, wurden als Rückfluss wieder in das Reaktionsgemisch zurückgeführt. Als kein Destillatanfall mehr zu beobachten war, wurde der Reaktionsfortschritt über eine GC-Analyse kontrolliert Die Reaktion wurde beendet, als der Triester >99 GC-Flächen% ausmachte, bezogen auf die Summe der Flächen des Edukts, Mono- Di- und Triesters.

Im Anschluss an die Reaktion, wurde der überschüssige Alkohol bei 160 °C unter Vakuum abdestilliert. Anschließend wurde die Säurezahl der Reaktionsmischung bestimmt und diese dann durch die Zugabe der 3-fachen stöchiometrischen Menge an 10%-NaOH neutralisiert. Als nächstes wurde die Mischung bei 160 °C mit Stickstoff für 2 h gestrippt. Im letzten Schritt wird das Produkt über einen 0,5 □m PTFE-Filter und Perlite als Filterhilfsmittel klar filtriert.

### Beispiel 1c - Herstellung von Cyclohexan-1,2,4-tripropionsäure-trisononylester (In-Tc)

Der nach Beispiel 1a) hergestellte Trimethylester (Me-Tc) wurde in einer typischen Labor Umesterungsapparatur wie in Beispiel 1b) mit Isononanol zum Produkt In-Tc umgeestert.

Zunächst wurden 1944 g Isononanol (INA, Hersteller Evonik Oxeno GmbH & Co. KG) und 1230 g M-Tc in der Umesterungsapparatur vorgelegt. Im Anschluss wurde die gesamte Apparatur mit Stickstoff gespült. Als nächstes wurden 2,03 g Tetra(n-butyl)titanat (TNBT) als Katalysator hinzugegeben. Danach wurde die Reaktion durch Aufheizen gestartet. Die Reaktionstemperatur betrug dabei 130 - 240°C, d. h. sie stieg während der Reaktion an. Methanol, das bei der Umesterung entstand, wurde über die Kolonne und Destillationsapparatur aus dem System entfernt (65°C Kopftemperatur). INA, dass in der Kolonne vom Methanol getrennt wurde, wurde als Rückfluss wieder in das Reaktionsgemisch zurückgeführt. Gelegentlich wurde leichtes Vakuum angelegt, um ausreichend Destillat zu erhalten. Als kein Destillatanfall mehr zu beobachten war, wurde der Reaktionsfortschritt über eine GC-Analyse kontrolliert. Die Reaktion war beendet, als der Triester >99 GC-Flächen% ausmachte, bezogen auf die Summe der Flächen des Edukts, Mono- Di- und Triesters.

Im Anschluss an die Reaktion, wurde der überschüssige Alkohol bei 180 °C unter Vakuum abdestilliert. Anschließend wurde die Säurezahl der Reaktionsmischung bestimmt und diese dann durch die Zugabe der 3-fachen stöchiometrischen Menge an 10%-NaOH neutralisiert. Als nächstes wurde die Mischung bei 180 °C mit Stickstoff für 2 h gestrippt. Im letzten Schritt wird das Produkt über einen 0,5 □m PTFE-Filter und Perlite als Filterhilfsmittel klar filtriert.

### Beispiel 2 - Herstellung von Plastisolen

Es wurden PVC-Plastisole hergestellt, wie sie beispielsweise zur Fertigung von Deckstrichfilmen für Fußbodenbeläge verwendet werden. Die Angaben in der Plastisolrezeptur sind jeweils in Gewichtsteilen (phr). Die Rezepturen der Polymerzusammensetzung ist in den Tabellen 1 und 2 aufgelistet.

**Tabelle 1: Übersicht über die hergestellten Plastisole (MbPe-Tc mit DBT bzw. DPT).**

| Plastisol | 1 | 2* | 3* |
|---|---|---|---|
| | | phr | phr |
| PVC (Vestolit^{®} P 1430 K 70 Ultra; Fa. Vestolit) | | 100 | 100 |
| MbPe-Tc (7:93) (gem. Beispiel 1b), (Mb: 2-Methylbutyl; Pe: *n*-Pentyl) | 50 | 33,33 | 33,33 |
| Eastman Effusion^{™} (Dibutylterephthalat, Fa. Eastman) | | 16,67 | |
| ELATUR^{®} DPT, (Di-pentylterephthalat, Fa. Evonik Oxeno & Co. KG) | | | 16,67 |
| epoxidiertes Sojabohnenöl als Co-Stabilisator (Edenol^{®} D81, Fa. Emery) | 3 | 3 | 3 |
| Thermostabilisator auf Ca/Zn-Basis (Reagens MBL 197/9 PF) | 2 | 2 | 2 |

| | | | |
|---|---|---|---|
| * = erfindungsgemäße Zusammensetzung phr = parts per hundred parts resin | | | |

**Tabelle 2: Übersicht über die hergestellten Plastisole (In-Tc/Me-Tc).**

| Plastisol | 4 | 5* | 6* | 7* | 8* |
|---|---|---|---|---|---|
| | | phr | phr | phr | phr |
| PVC (Vestolit^{®} P 1430 K 70 Ultra; Fa. Vestolit) | 100 | 100 | 100 | 100 | 100 |
| In-Tc (In: Isononyl) (gem. Beispiel 1c) | 50 | 33,33 | 16,67 | 33,33 | 16,67 |
| Eastman Effusion^{™} (Dibutylterephthalat, Fa. Eastman) | | 16,67 | 33,33 | | |
| ELATUR^{®} DPT, (Di-pentylterephthalat, Fa. Evonik Oxeno & Co. KG) | | | | 16,67 | 33,33 |
| epoxidiertes Sojabohnenöl als Co-Stabilisator (Edenol^{®} D81, Fa. Emery) | 3 | 3 | 3 | 3 | 3 |
| Thermostabilisator auf Ca/Zn-Basis (Reagens MBL 197/9 PF) | 2 | 2 | 2 | 2 | 2 |

| | | | | | |
|---|---|---|---|---|---|
| * = erfindungsgemäße Zusammensetzung | | | | | |

Zuerst wurden die flüssigen und dann die pulverförmigen Bestandteile in einen PE-Becher eingewogen. Von Hand wurde die Mischung mit einem Spatel so eingerührt, dass kein unbenetztes Pulver mehr vorhanden war. Der verschlossene Becher wurde in die im Speedmixer befindliche Halterung gestellt und vermischt sowie entlüftet. Nach Beenden der Vermischung wurde die Temperatur des Plastisols mit Hilfe eines IR-Thermometers gemessen. Danach wurde das Plastisol sofort für weitere Untersuchungen in einem Klimaschrank bei 25,0 °C temperiert.

### Beispiel 3 - Gelierung der Plastisole

Die Untersuchung des Gelierverhaltens der Plastisole aus Beispiel 2 wurde mittels Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| | |
|---|---|
| Modus: | Temperatur-Gradient |
| Start-Temperatur: | 25 °C |
| End-Temperatur: | 180 °C |
| Heiz/Kühlrate: | 5 °C/min |
| Oszillations-Frequenz: | 4 bis 0,1 Hz Rampe logarithmisch |
| Kreisfrequenz Omega: | 10 s⁻¹ |
| Anzahl Messpunkte: | 63 |
| Messpunktdauer: | 0,5 min |
| Automatische Spaltnachführung F: | 0 N |
| Konstante Messpunktdauer | |
| Spaltweite | 0,5 mm |

### Durchführung der Messung

Auf die untere Messsystemplatte wurden mit dem Spatel einige Gramm der zu messenden Paste luftblasenfrei aufgetragen. Dabei wurde darauf geachtet, dass nach dem Zusammenfahren des Messsystems etwas Paste gleichmäßig aus dem Messsystem herausquellen konnte (nicht mehr als 6 mm rundum). Der Überschuss wurde mittels Spatel entfernt. Anschließend wurde die Temperierhaube über der Probe positioniert und die Messung gestartet. Bestimmt wurde die komplexe Viskosität der Paste nach 24 Stunden (Lagerung der Paste bei 25 °C in einem Temperierschrank der Fa. Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Pastenviskosität von 1000 Pa*s verwendet. Die erhaltenen Ergebnisse sind in Tabelle 3 aufgeführt.

**Tabelle 3: Gelierung der Plastisole nach 24 Stunden, Temperatur in °C bei Erreichen einer Pastenviskosität von 1000 Pa*s.**

| Rezeptur | Weichmacherzusammensetzung nach Tabellen 1 und 2 | Geliertemperatur [°C] |
|---|---|---|
| 1 | MbPe-Tc | 77,3 |
| 2* | MbPe-Tc : DBT (2:1) | 69,8 |
| 3* | MbPe-Tc : DPT (2:1) | 72,7 |
| 4 | In-Tc | 135,9 |
| 5* | In-Tc : DBT (2:1) | 82,8 |
| 6* | In-Tc : DBT (1:2) | 68,0 |
| 7* | In-Tc : DPT (2:1) | 87,7 |
| 8* | In-Tc : DPT (1:2) | 74,8 |

| | | |
|---|---|---|
| * Versuch mit erfindungsgemäßer Weichmacherzusammensetzung | | |

Die Zugabe von DBT oder DPT zu C4- bis C9-Estern der 1,2,4-Cyclohexantricarbonsäure sorgt für eine deutlich beschleunigte Gelierung im Vergleich zu den C4- bis C9-Estern der 1,2,4-Cyclohexantricarbonsäure ohne zusätzlichen Weichmacher.

### Beispiel 4 - Messung der Plastisolviskositäten

Die Messung der Viskosität der in Beispiel 2 hergestellten Plastisole wurde mit einem Rheometer Physica MCR 101 (Firma Anton Paar Germany GmbH) mit Hilfe der zugehörigen Software durchgeführt, wobei der Rotationsmodus und das Messsystem CC27 verwendet wurden.

Während der Messung wurden folgende Punkte angesteuert:
- Vorscherung bei 100 s-1 für einen Zeitraum von 60 Sekunden, bei der keine Messwerte aufgenommen wurden
- Scherraten-Abwärtsrampe von 200 s-1 bis 0,1 s-1. Es wurden 30 Messpunkte aufgenommen mit einer Messpunktdauer von je 10 Sekunden.

Die Messungen wurden bei Raumtemperatur durchgeführt. Die Ergebnisse der Viskositätsmessungen der in Beispiel 2 hergestellten Plastisole mit den jeweils angegebenen Weichmachern bzw. Weichmachermischungen sind in Tabelle 4 gezeigt.

**Tabelle 4: Plastisolviskositäten und Glasübergangstemperaturen**

| Rezeptur | Weichmacherzusammensetzung nach Tabellen 1 und 2 | Pastenviskosität nach 24 Stunden bei 200 s⁻¹ / Pa*s | T_{G} in °C |
|---|---|---|---|
| 1* | MbPe-Tc | 7,95 | -29 |
| 2* | MbPe-Tc : DBT (2:1) | 6,43 | -33 |
| 3* | MbPe-Tc : DPT (2:1) | 6,72 | -33 |
| 4* | In-Tc | >17^{a} | -44 |
| 5* | In-Tc : DBT (2:1) | 7,88 | -41 |
| 6* | In-Tc : DBT (1:2) | 5,86 | -35 |
| 7* | In-Tc : DPT (2:1) | 9,52 | -41 |
| 8* | In-Tc : DPT (1:2) | 6,5 | -37 |

| | | | |
|---|---|---|---|
| a: Messung bei Scherraten > 137 s⁻¹ aufgrund hoher Viskosität abgebrochen. | | | |

Die Zugabe von DBT oder DPT zu C4- bis C9-Estern der 1,2,4-Cyclohexantricarbonsäure sorgt für eine deutlich Verringerung der Pastenviskosität im Vergleich zu den C4- bis C9-Estern der 1,2,4-Cyclohexantricarbonsäure ohne zusätzlichen Weichmacher. Zudem wird die Glasübergangstemperatur durch die Zugabe von DBT und DPT weiter abgesenkt.

## Patentansprüche

1. Weichmacherzusammensetzung enthaltend eine Verbindung der Formel (1) 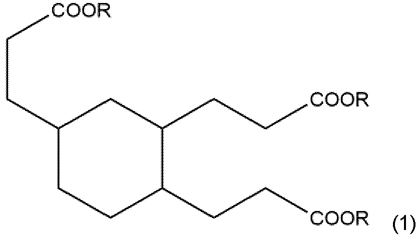 wobei die drei Alkylgruppen R jeweils 4 bis 9 Kohlenstoffatome aufweisen, und Dibutylterephthalat oder Dipentylterephthalat.

2. Weichmacherzusammensetzung nach Anspruch 1, wobei die drei Alkylgruppen R jeweils unabhängig voneinander aus der Gruppe, bestehend aus einer normalen Butylgruppe, einer Isobutylgruppe, einer normalen Pentylgruppe, einer 2-Methylbutylgruppe, einer 3-Methylbutylgruppe, einer normalen Hexylgruppe, einer Isohexylgruppe, einer normalen Heptylgruppe, einer Isoheptylgruppe, einer normalen Ocylgruppe, einer Isooctylgruppe, einer normalen Nonylgruppe oder einer Isononylgruppe, ausgewählt werden.

3. Weichmacherzusammensetzung nach Anspruch 1 oder 2, wobei die drei Alkylgruppen R der Verbindung nach Formel (1) die gleiche Anzahl an Kohlenstoffatomen aufweisen.

4. Weichmacherzusammensetzung nach einem der vorhergehenden Ansprüche, wobei die Verbindung nach Formel (1) ein Tripentylester der 1,2,4-Cyclohexantripropionsäure oder ein Triisononylester der 1,2,4-Cyclohexantripropionsäure ist.

5. Weichmacherzusammensetzung nach einem der vorhergehenden Ansprüche, wobei die Verbindung nach Formel (1) und Dibutylterephthalat oder Dipentylterephthalat in einem Gewichtsverhältnis (Verbindung nach Formel (1) : Dibutylterephthalat oder Dipentylterephthalat) von 1 : 99 bis 99 : 1, vorzugsweise 10 : 90 bis 90 : 10, besonders bevorzugt 30 : 70 bis 70 : 30 in der Weichmacherzusammensetzung vorhanden sind.

6. Weichmacherzusammensetzung nach einem der vorhergehenden Ansprüche, wobei die Weichmacherzusammensetzung zusätzlich ein epoxidiertes Öl oder einen epoxidierten Alkylester einer Fettsäure enthält.

7. Weichmacherzusammensetzung nach Anspruch 6, wobei das epoxidierte Öl aus der Gruppe bestehend aus epoxidiertem Sojabohnenöl, epoxidiertem Rizinusöl, epoxidiertem Leinöl, epoxidiertem Palmöl, epoxidiertem Stearat, epoxidiertem Oleat, epoxidiertem Tallöl, epoxidiertem Linoleat und Mischungen davon ausgewählt wird.

8. Weichmacherzusammensetzung nach Anspruch 7, wobei das epoxidierte Öl epoxidiertes Sojabohnenöl oder epoxidiertes Leinöl, vorzugsweise epoxidiertes Sojabohnenöl ist.

9. Weichmacherzusammensetzung nach einem der Ansprüche 6 bis 8, wobei das epoxidierte Öl in einer Menge von 1 bis 150 Gewichtsteilen bezogen auf 100 Gewichtsteile der Gesamtsumme aus der Verbindung gemäß Formel (1) und Dibutylterephthalat oder Dipentylterephthalat in der Weichmacherzusammensetzung vorhanden ist.

10. Weichmacherzusammensetzung nach einem der vorherigen Ansprüche, wobei die Zusammensetzung zusätzlich noch einen weiteren Weichmacher aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten mit Ausnahme von Dibutylterephthalat oder Dipentylterephthalat, Isophthalaten, Trimellitaten, Cyclohexandicarboxylaten und oligomeren oder polymeren Estern auf Basis von Adipin-, Bernstein- oder Sebacinsäure, ausgewählt werden.

11. Kunststoffzusammensetzung, umfassend die Weichmacherzusammensetzung nach einem der Ansprüche 1 bis 10 und einen Kunststoff.

12. Kunststoffzusammensetzung nach Anspruch 11, wobei der Anteil der Weichmacherzusammensetzung 5 bis 150 Gewichtsteile pro 100 Gewichtsteile Kunststoff beträgt.

13. Kunststoffzusammensetzung nach Anspruch 11 oder 12, wobei der Kunststoff aus der Gruppe, bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat, ausgewählt wird.

14. Kunststoffzusammensetzung nach Anspruch 13, wobei der Kunststoff Polyvinylchlorid (PVC) ist.

15. Verwendung der Kunststoffzusammensetzung nach einem der Ansprüche 11 bis 14 in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

## Claims

1. Plasticizer composition comprising a compound of the formula (1) 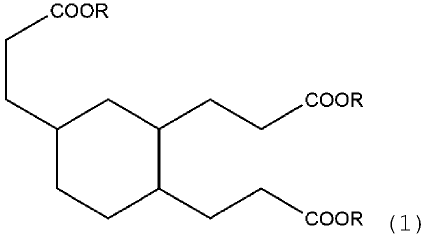 where the three alkyl groups R each have 4 to 9 carbon atoms, and dibutyl terephthalate or dipentyl terephthalate.

2. Plasticizer composition according to Claim 1, wherein the three alkyl groups R are each independently selected from the group consisting of a normal butyl group, an isobutyl group, a normal pentyl group, a 2-methylbutyl group, a 3-methylbutyl group, a normal hexyl group, an isohexyl group, a normal heptyl group, an isoheptyl group, a normal octyl group, an isooctyl group, a normal nonyl group or an isononyl group.

3. Plasticizer composition according to Claim 1 or 2, wherein the three alkyl groups R of the compound of formula (1) have the same number of carbon atoms.

4. Plasticizer composition according to any of the preceding claims, wherein the compound of formula (1) is a tripentyl ester of cyclohexane-1,2,4-tripropionic acid or a triisononyl ester of cyclohexane-1,2,4-tripropionic acid.

5. Plasticizer composition according to any of the preceding claims, wherein the compound of formula (1) and dibutyl terephthalate or dipentyl terephthalate are present in the plasticizer composition in a weight ratio (compound of formula (1) : dibutyl terephthalate or dipentyl terephthalate) of 1 : 99 to 99 : 1, preferably 10 : 90 to 90 : 10, more preferably 30 : 70 to 70 : 30.

6. Plasticizer composition according to any of the preceding claims, wherein the plasticizer composition additionally comprises an epoxidized oil or an epoxidized alkyl ester of a fatty acid.

7. Plasticizer composition according to Claim 6, wherein the epoxidized oil is selected from the group consisting of epoxidized soybean oil, epoxidized castor oil, epoxidized linseed oil, epoxidized palm oil, epoxidized stearate, epoxidized oleate, epoxidized tall oil, epoxidized linoleate and mixtures thereof.

8. Plasticizer composition according to Claim 7, wherein the epoxidized oil is epoxidized soybean oil or epoxidized linseed oil, preferably epoxidized soybean oil.

9. Plasticizer composition according to any of Claims 6 to 8, wherein the epoxidized oil is present in the plasticizer composition in an amount of 1 to 150 parts by weight based on 100 parts by weight of the sum total of the compound of formula (1) and dibutyl terephthalate or dipentyl terephthalate.

10. Plasticizer composition according to any of the preceding claims, wherein the composition additionally a further plasticizer from the group consisting of adipates, benzoates, for example monobenzoates or glycol dibenzoates, chlorinated hydrocarbons (called chloroparaffins), citrates, epoxidized fatty acid esters, epoxidized vegetable oils, epoxidized acylated glycerides, furandicarboxylates, phosphates, succinates, sulfonamides, sulfonates, terephthalates excluding dibutyl terephthalate or dipentyl terephthalate, isophthalates, trimellitates, cyclohexanedicarboxylates and oligomeric or polymeric esters based on adipic acid, succinic acid or sebacic acid are selected.

11. Plastics composition comprising the plasticizer composition according to any of Claims 1 to 10 and a plastic.

12. Plastics composition according to Claim 11, wherein the proportion of the plasticizer composition is 5 to 150 parts by weight per 100 parts by weight of plastic.

13. Plastics composition according to Claim 11 or 12, wherein the plastic is selected from the group consisting of polyvinylchloride (PVC), polyalkylmethacrylate (PAMA), polyvinylbutyral (PVB), polyurethane, polysulfides, polylactic acid (PLA), polyhydroxybutyral (PHB), nitrocellulose and copolymers of vinyl chloride with vinyl acetate or with butyl acrylate.

14. Plastics composition according to Claim 13, wherein the plastic is polyvinylchloride (PVC).

15. Use of the plastics composition according to any of Claims 11 to 14 in adhesives, sealing compounds, coating compounds, varnishes, paints, plastisols, foams, imitation leathers, floor coverings, especially the top layer and foam layer, roofing membranes, underbody protection, fabric coatings, cables, wire insulation, hoses, extruded articles, films, in the automotive interior sector, in wallpaper, inks, toys, contact sheets, food packaging or medical articles, especially in tubes or blood bags.

## Revendications

1. Composition plastifiante, contenant un composé de formule (1) 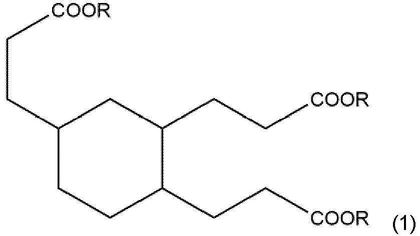 dans laquelle les trois groupes alkyle R présentent chacun 4 à 9 atomes de carbone, et du téréphtalate de dibutyle ou du téréphtalate de dipentyle.

2. Composition plastifiante selon la revendication 1, dans laquelle les trois groupes alkyle R sont choisis chacun indépendamment les uns des autres dans le groupe constitué par un groupe butyle normal, un groupe isobutyle, un groupe pentyle normal, un groupe 2-méthylbutyle, un groupe 3-méthylbutyle, un groupe hexyle normal, un groupe isohexyle, un groupe heptyle normal, un groupe isoheptyle, un groupe octyle normal, un groupe isooctyle, un groupe nonyle normal, ou un groupe isonyle.

3. Composition plastifiante selon la revendication 1 ou 2, dans laquelle les trois groupes alkyle R du composé de formule (1) présentent le même nombre d'atomes de carbone.

4. Composition plastifiante selon l'une quelconque des revendications précédentes, dans laquelle le composé selon la formule (1) est un ester tripentylique de l'acide 1,2,4-cyclohexantripropionique ou un ester triisonylique de l'acide 1,2,4-cyclohexantripropionique.

5. Composition plastifiante selon l'une quelconque des revendications précédentes, dans laquelle le composé selon la formule (1) et le téréphtalate de dibutyle ou le téréphtalate de dipentyle sont présents selon un rapport pondéral (composé selon la formule (1) : téréphtalate de dibutyle ou téréphtalate de dipentyle) de 1 : 99 à 99 : 1, de préférence de 10 : 90 à 90 : 10, de manière particulièrement préférée de 30 : 70 à 70 : 30 dans la composition plastifiante.

6. Composition plastifiante selon l'une quelconque des revendications précédentes, la composition plastifiante contenant en plus une huile époxydée ou un ester alkylique époxydé d'un acide gras.

7. Composition plastifiante selon la revendication 6, l'huile époxydée étant choisie dans le groupe constitué par l'huile de germes de soja époxydée, l'huile de ricin époxydée, l'huile de lin époxydée, l'huile de palme époxydée, le stéarate époxydé, l'oléate époxydé, l'huile de tall époxydée, le linoléate époxydé et leurs mélanges.

8. Composition plastifiante selon la revendication 7, l'huile époxydée étant l'huile de germes de soja époxydée ou l'huile de lin époxydée, de préférence l'huile de germes de soja époxydée.

9. Composition plastifiante selon l'une quelconque des revendications 6 à 8, l'huile époxydée étant présente en une quantité de 1 à 150 parties en poids par rapport à 100 parties en poids de la quantité totale du composé selon la formule (1) et du téréphtalate de dibutyle ou du téréphtalate de dipentyle dans la composition plastifiante.

10. Composition plastifiante selon l'une quelconque des revendications précédentes, la composition et en plus encore un autre plastifiant du groupe constitué par les adipates, les benzoates, par exemple les monobenzoates ou les dibenzoates de glycol, les hydrocarbures chlorés (appelés chloroparaffines), les citrates, les esters d'acide gras époxydés, les huiles végétales epoxydées, les glycérides acylés époxydés, les furannedicarboxylates, les phosphates, les succinates, les sulfonamides, les sulfonates, les téréphtalates à l'exception du téréphtalate de dibutyle ou du téréphtalate de dipentyle, les isophtalates, les trimellitates, les cyclohexanedicarboxylates et les esters oligomères ou polymères à base d'acide adipique, succinique ou sébacique sont choisis.

11. Composition de matériau synthétique comprenant la composition plastifiante selon l'une quelconque des revendications 1 à 10 et un matériau synthétique.

12. Composition de matériau synthétique selon la revendication 11, la proportion de composition plastifiante représentant 5 à 150 parties en poids par 100 parties en poids de matériau synthétique.

13. Composition de matériau synthétique selon la revendication 11 ou 12, le matériau synthétique étant choisi dans le groupe constitué par le poly(chlorure de vinyle) (PVC), le poly(méthacrylate d'alkyle) (PAMA), le polyvinylbutyral (PVB), le polyuréthane, le polysulfure, le poly(acide lactique) (PLA), le polyhydroxybutyral (PHB), la nitrocellulose et les copolymères du chlorure de vinyle avec de l'acétate de vinyle ou de l'acrylate de butyle.

14. Composition de matériau synthétique selon la revendication 13, le matériau synthétique étant le poly(chlorure de vinyle) (PVC).

15. Utilisation de la composition de matériau synthétique selon l'une quelconque des revendications 11 à 14 dans les adhésifs, les masses d'étanchéité, les masses de revêtement, les vernis, les peintures, les plastisols, les mousses, le cuir synthétique, les revêtements de sol, en particulier la couche de recouvrement et la couche de mousse, les membranes de toiture, la protection de bas de caisse, les revêtements de tissu, les câbles, les isolations de fil, les flexibles, les articles extrudés, les feuilles, à l'intérieur de véhicules, dans les tapis, les encres, les jouets, les films de contact, les emballages alimentaires ou les articles médicaux, en particulier dans les flexibles ou les poches à sang.
